# EUROPEAN PATENT APPLICATION

(11) **EP 2 062 911 A1**
(43) Date of publication of application: **27.05.2009**
(21) Application number: 07121522.2
(22) Date of filing: 26.11.2007
(51) Int. Cl.: C07K 1/14

(54) **Selective enrichment of post-translationally modified proteins**

(71) Applicant: Koninklijke Philips Electronics N.V., 5621 BA Eindhoven (NL)
(72) Inventor: The designation of the inventor has not yet been filed
(74) Representative: Plünnecke, Ingo

(57) **Abstract**

The present invention relates to the selective enrichment of post-translationally modified proteins and/or peptides from complex samples. In particular, the invention relates to methods for the separation of phospho-proteins and/or -peptides and/or for the discrimination between different subsets of phospho-proteins and/or -peptides.

## Description

### SUBJECT OF THE INVENTION

The present invention relates to the selective enrichment of post-translationally modified proteins and/or peptides from complex samples. In particular, the invention relates to methods for the separation of phospho-proteins and/or -peptides and/or for the discrimination between different subsets of phospho-proteins and/or -peptides.

### BACKGROUND OF THE INVENTION

The identification, separation, and analysis of particular proteins or subsets of proteins from complex samples are invaluable for unraveling how biological processes occur at a molecular level or to which degree proteins differ among various cell types or between physiological states.

A major challenge in modem biology is directed to the understanding of the expression, function, and regulation of the entire set of proteins encoded by an organism, a technical field commonly known as proteomics. However, such a task is beyond the capabilities of any current single analytical methods. Thus, due to the methodological constraints proteome analysis relies not only to methods for identifying and quantifying proteins but - to a considerable extent - also on methods allowing their accurate and reliable separation according to their structural and/or functional properties, with these subsets being then better accessible to further analysis.

The proteome is of dynamic nature, with alterations in protein synthesis, activation, and/or post-translational modification in response to external stimuli or alterations in the cellular environment. Therefore, the proteome's inherent complexity exceeds that of the genome or the transcriptome, the mRNA complement of a cell.

An important facet of protein analysis in general and proteomics in particular relates to the possibility to study post-translational protein modifications which can affect activity and binding of a protein and alter its role within the cell (cf., e.g., Pandey, A. and Mann, M. (2000) Nature 405, 837-846). For example, the (reversible) phosphorylation of proteins is crucial for the regulation of many signal transduction cascades such as G-protein-coupled receptor signaling or phosphotyrosine kinase signaling, whereas an ubiquitination *inter alia* labels proteins for degradation.

One of the unique features of proteomics is that post-translational modifications can be investigated at a more global level, thus allowing the analysis of the entire subset of proteins comprising a particular modification. The expressed products of a single gene represent a protein population that may contain large amounts of micro-heterogeneity, each different state (i.e. analogous proteins differing in the number of post-translationally modified amino acid residues) adding a large amount of diversity to the expression profile of that protein.

Currently, there are several techniques available, for example mass spectrometry, which can in principle distinguish between analogous proteins or peptides due to the presence or absence of a specific modification. However, these changes are frequently not observed in global proteomic studies due to a limited sensitivity of detection. Thus, in order to study a specific post-translational modification, it would be helpful to enrich a sample for that modification, usually by some form of affinity purification, and/or to separate the enriched subset of modified proteins from that sample. However, the available methods are generally hampered by the requirement to label the proteins with appropriate affinity tags or the need to use specific antibodies or other reagents which might interfere with further analyses. Therefore, such methods based on affinity purification are particularly not suitable for processing multiple samples in parallel.

In addition, it is generally cumbersome to distinguish different subsets of proteins and/or peptides bearing a particular modification (e.g., tyrosine-phosphorylated and serine/threonin-phosphorylated proteins) based on affinity purification protocols.

The study of protein phosphorylation has grown exponentially in recent years as researchers from various disciplines have come to realize that key cellular functions are regulated by the reversible phosphorylation and dephosphorylation of proteins on serine, threonine and tyrosine residues. At present, only a subset of the known eukaryotic protein kinases and protein phosphatases has been characterized with respect to biological function and protein substrate specificity. To understand more about protein phosphorylation and dephosphorylation, it is necessary to identify the specific amino acid residues that become phosphorylated, because identification of these sites in proteins may reveal which protein kinase regulates the protein and thereby help elucidate the biological function and significance of novel phospho-proteins

Therefore, it is a continuing need for methods allowing the selective enrichment ofpost-translationally modified proteins and/or peptides from complex samples. In particular, it would be desirable to provide methods for the separation and/or discrimination of phospho-proteins and/or -peptides not only with high sensitivity but also without the requirement of specific reagents.

### OBJECT AND SUMMARY OF THE INVENTION

It is an objective of the present invention to provide novel approaches for the selective enrichment of post-translationally modified peptides and/or proteins, in particular of phospho-proteins and/or -peptides, from complex samples.

It is a further objective of the present invention to provide methods that allow separating post-translationally modified proteins and/or peptides from their unmodified counterparts and/or to discriminate between different subsets of these modified proteins and/or peptides.

These objectives as well as others which will become apparent from the ensuing description are attained by the subject matter of the independent claims. Some of the preferred embodiments of the present invention are defined by the subject matter of the dependent claims.

In one embodiment, the present invention relates to a method for the selective enrichment and/or separation of post-translationally modified proteinaceous molecules, comprising at least the steps of:
(a) fractionating the proteinaceous molecules;
(b) removing or altering a specific post-translational modification from at least a first subset of the proteinaceous molecules;
(c) re-fractionating the proteinaceous molecules obtained in step (b);
(d) comparing the fractionation patterns obtained in steps (a) and (c); and
(e) analyzing the at least first subset of proteinaceous molecules modified in step
(b) based on the results obtained in step (d).

In a specific embodiment, the method further comprises cleaving the proteinaceous molecules into peptides prior to subjecting them to fractionation.

In a further specific embodiment, the fractionation/re-fractionation of the proteinaceous proteins is performed by isoelectric focusing.

Preferably, the methods according to the present invention are used to investigate phospho-proteins and/or -peptides. Thus, in a typical embodiment the post-translational modification of the at least first subset of the proteinaceous molecules that is to be removed or altered is a phosphate-group.

In a preferred embodiment, the at least first subset of proteinaceous molecules relates to serine- and threonine-phosphorylated proteinaceous molecules.

Preferably, the phosphate-group is removed from at least first subset of proteinaceous molecules chemically via β-elimination.

In another embodiment of the inventive method, after performing step (e) the remaining subset/s of proteinaceous molecules is/are subjected to another cycle of steps (a) to (e), and wherein step (b) comprises removing or altering a specific post-translational modification from at least a second subset of the proteinaceous molecules.

Typically, the post-translational modification from the at least second subset of the proteinaceous molecules that is to be removed or altered is a phosphate-group. Preferably, the phosphate-group is removed from said at least second subset of proteinaceous molecules enzymatically via phosphatases.

In a further preferred embodiment, the at least second subset of proteinaceous molecules relates to tyrosine-phosphorylated proteinaceous molecules.

Preferably, the phosphate-group is removed from at least second subset of proteinaceous molecules enzymatically via phosphatases.

The method according to the invention, as described herein, may be used for the qualitative and/or quantitative determination of phospho-proteins/peptides. In preferred embodiments, the determination is performed via mass spectrometry.

Other embodiments of the present invention will become apparent from the detailed description hereinafter.

### FIGURE LEGENDS

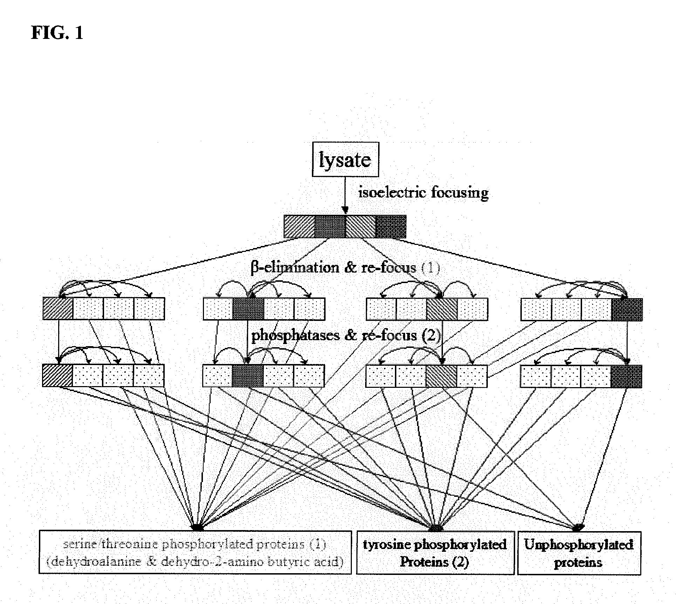

Fig. 1 depicts a schematic illustration of the application of a preferred embodiment of the invention to the selective enrichment of phospho-proteins. A sample of intact proteins is fractionated by isoelectric focusing (IEF). All individual fractions are collected and individually treated with a reaction mixture for β-elimination that specifically removes the serine- and threonine-phosphate groups from the phospho-proteins. All fractions are then individually re-fractionated by IEF. Proteins sorted to the same fraction as after the first round of IEF have not undergone any modification and are thus tyrosine-phosphorylated or unphosphorylated proteins. However, proteins sorted to a different fraction after re-fractionation have undergone β-elimination and are thus the serine- or threonine-phosphorylated proteins. After separation the latter subsets of phospho-proteins the remaining fractions are subjected to another round of treatment using phosphatases for enzymatically removing phosphate groups from tyrosine-phosphorylated proteins which are then separated by another round of re-fractionating by IEF.

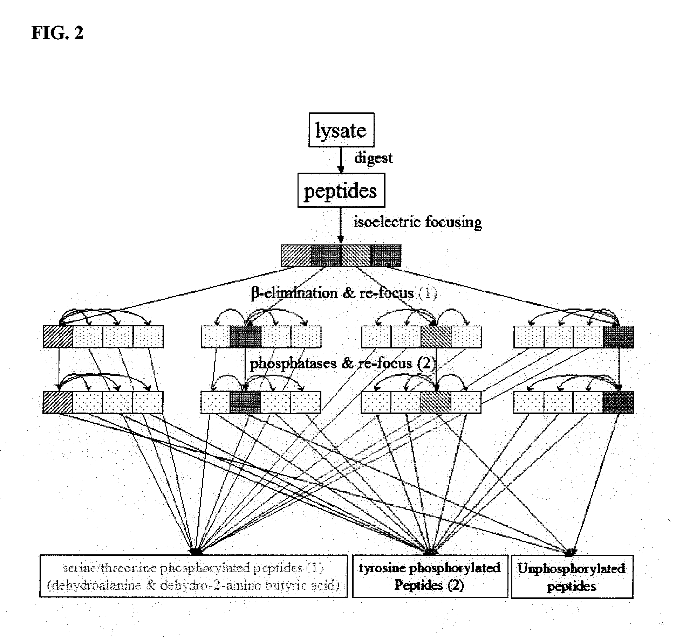

Fig. 2 depicts a schematic illustration of the application of a preferred embodiment of the invention to the selective enrichment of phospho-peptides. A sample of intact proteins is digested into peptides and subjected to the same experimental protocol as described in Fig. 1.

### DETAILED DESCRIPTION OF THE INVENTION

The present invention is based on the unexpected finding that combining protein fractionation with specific chemical and/or enzymatic reactions targeting the post-translational protein modification to be analyzed allows the rapid and highly selective enrichment and/or separation of said modified proteins from a complex sample. Furthermore, by adapting the reaction conditions the same method is also appropriate to discriminate between different subsets of proteins bearing a particular post-translational modification.

The present invention illustratively described in the following may suitably be practiced in the absence of any element or elements, limitation or limitations, not specifically disclosed herein.

The present invention will be described with respect to particular embodiments and with reference to certain drawings but the invention is not limited thereto but only by the claims.

The drawings described are only schematic and are non-limiting. In the drawings, the size of some of the elements may be exaggerated and not drawn on scale for illustrative purposes.

Where the term "comprising" is used in the present description and claims, it does not exclude other elements or steps. For the purposes of the present invention, the term "consisting of" is considered to be a preferred embodiment of the term "comprising of". If hereinafter a group is defined to comprise at least a certain number of embodiments, this is also to be understood to disclose a group which preferably consists only of these embodiments.

Where an indefinite or definite article is used when referring to a singular noun e.g. "a" or "an", "the", this includes a plural of that noun unless something else is specifically stated.

The term "about" in the context of the present invention denotes an interval of accuracy that the person skilled in the art will understand to still ensure the technical effect of the feature in question. The term typically indicates deviation from the indicated numerical value of ± 10%, and preferably 5%.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used.

In one embodiment, the present invention relates to a method for the selective enrichment and/or separation of post-translationally modified proteinaceous molecules, comprising at least the steps of:
(a) fractionating the proteinaceous molecules;
(b) removing or altering a specific post-translational modification from at least a first subset of the proteinaceous molecules;
(c) re-fractionating the proteinaceous molecules obtained in step (b);
(d) comparing the fractionation patterns obtained in steps (a) and (c); and
(e) analyzing the at least first subset of proteinaceous molecules modified in step
(b) based on the results obtained in step (d).

The term "proteinaceous molecules", as used herein, refers to any naturally occurring or synthetic (e.g., generated by chemical synthesis or recombinant DNA technology) macromolecules comprising a plurality of natural or modified amino acids connected via a peptide bond.

The length of such a proteinaceous molecule may vary from two to several thousand amino acids (the term thus also includes what is generally referred to as oligopeptides). Typically, the term "proteinaceous molecules" relates to proteins having a length of more than 20 amino acids. Thus, proteins to be analyzed in the present invention may have a length from about 30 to about 2500 amino acids, from about 50 to about 1000 amino acids or from about 100 to about 1000 amino acids.

Particularly included within this scope are proteinaceous molecules comprising one or more amino acids which are modified by *in vivo* post-translational modifications (e.g., phosphorylation) and/or comprising one or more amino acids which have been modified *in vitro* with protein modifying agents (e.g. alkylating agents).

The term "peptide", as used herein, refers to any fragments of the above "proteinaceous molecules" that are obtained after cleavage of one or more peptide bonds. A peptide as used in the present invention is not limited in any way with regard to its size or nature. Typically, peptides to be analyzed in the present invention may have a length from about 2 to about 20 amino acids, from about 3 to about 18 amino acids or from about 5 to about 15 amino acids.

The term "post-translational modification", as used herein, denotes any type of chemical modification of a proteinaceous molecule according to the invention that takes place after completion of protein translation. Examples of such modifications include *inter alia* phosphorylation, ubiquitinylation, acetylation, glycosylation, alkylation, isoprenylation, and lipoylation. In preferred embodiments, the post-translational modification is phosphorylation. The term is also to be understood not to be limited with regard to the numbers and/of types of post-translational modifications being comprised in a proteinaceous molecules. Thus, a given protein may comprise in its sequence, for example, two or more phosphorylated amino acids or one or more phosphorylated amino acids and one or more ubiquitinylated amino acid residues.

The proteinaceous molecules are enriched and/or separated by means of the inventive method from a sample comprising such molecules, preferably from a biological sample. The term "sample", as used herein, is not intended to necessarily include or exclude any processing steps prior to the performing of the methods of the invention. The samples can be unprocessed ("crude") samples, extracted protein fractions, purified protein fractions, and the like. For example, the samples employed may be pre-processed by immunodepletion of one or more subsets of abundant proteins. Suitable samples include samples of prokaryotic (e.g., bacterial, viral samples) or eukaryotic origin (e.g., fungal, yeast, plant, invertebrate, mammalian, and particularly human samples).

The terms "at least a first subset of the proteinaceous molecules" and " at least a second subset of the proteinaceous molecules", as used herein, are to be understood in such a way that they may relate to the totality of proteinaceous molecules present in a given sample or a particular part thereof.

The terms "fractionation" and "re-fractionaction", as used herein, refer to any type of separation process in which the proteinaceous molecules present in a sample in a certain quantity are divided (i.e. sorted) up in a large number of smaller quantities (i.e. fractions) according to any differences in their physico-chemical properties such as the molecular mass, their size, and their overall net charge. A common trait in fractionations is the need to find an optimum between the amount of fractions collected and the desired purity in each fraction. Fractionation makes it possible to isolate more than two components in a mixture in a single run. This property sets it apart from other separation techniques. There are several methods for fractionating proteins and/or peptides well established in the art including classical SDS polyacrylamide gel electrophoresis (SDS-PAGE), two-dimensional gel electrophoresis, size-exclusion chromatography, (two-dimensional) liquid chromatography, and isoelectric focusing, with the latter one being particularly preferred. Methods for analysis, particularly visualization, of the proteinaceous molecules after fractionation has taken place are well established in the art.

Isoelectric focusing is a technique for separating different molecules by their electric charge differences. It is a type of zone electrophoresis, usually performed in a gel (e.g., an agarose gel or, preferably, polyacrylamide gel) or in liquid phase, that takes advantage of the fact that a molecule's charge changes with the pH of its surroundings. The molecules to be focused are distributed over a medium that has a pH gradient. An electric current is passed through the medium, creating a "positive" anode and "negative" cathode end. Negatively charged molecules migrate through the pH gradient in the medium toward the "positive" end while positively charged molecules move toward the "negative" end. As a particle moves towards the pole opposite of its charge it moves through the changing pH gradient until it reaches a point in which the pH of that molecules isoelectric point (pI) is reached. At this point the molecule no longer has a net electric charge (due to the protonation or deprotonation of the associated functional groups) and as such will not proceed any further within the gel. Isoelectric focusing can resolve proteins that differ in pI value by as little as 0.01.

Isoelectric focusing is usually the first step in two-dimensional gel electrophoresis, in which proteins are first separated by their pI and then further separated by molecular weight through standard SDS-PAGE.

The term "re-fractionating", as used herein, also denotes that the method of the invention may be performed with a single type of fractionation method, that is, the proteinaceous molecules are fractionated before and after removing and/or altering a specific post-translational modification by applying the same method, preferably isoelectric focusing. However, depending on the particular protocol it may also be possible to use different methods, e.g. isoelectric focusing and classical SDS-PAGE.

Other methods of fractionation/re-fractionation and combinations thereof may also be envisaged. Thus, fractionation/re-fractionation may rely on ion exchange chromatography, side exclusion chromatography, hydrophobic interaction chromatography, reversed-phase chromatography and/or (immuno)affinity chromatography.

In preferred embodiments, the method further comprises cleaving the proteinaceous molecules into peptides prior to subjecting them to fractionation. Such cleaving of the proteinaceous molecules may either be achieved chemically (e.g., via acid or base treatment employing chemicals such as cyanogen bromide, 2-(2'-nitro-phenylsulfonyl)-3-methyl-3-bromo-indolenine (BNPS), formic acid, hydroxylamine, iodobenzoic acid, and 2-nitro-5-thiocyanobenzoid acid) or enzymatically via proteases (including *inter alia* trypsin, pepsin, thrombin, papain, and proteinase K) well established in the art.

In particular preferred embodiments of the invention, the post-translational modification of the at least first subset of the proteinaceous molecules that is to be removed or altered is a phosphate-group. In other words, the method according to the invention is preferably used to analyze phospho-proteins and/or -peptides. The term "removing", as used herein, refers to the complete elimination of the modification, for example by chemical cleavage or enzyme action (see also the discussion below). The term "altering", as used herein, denotes any modification of this post-translational modification resulting in a change in the physico-chemical properties of the proteinaceous molecules that allows a discrimination between the variants bearing the original and the altered post-translational modifications. Examples of such alterations include *inter alia* the cleavage of one or more chemical bonds within the functional group representing the modification without removing the modification from the amino acid backbone as well as the conjugation of the modification with any moiety, thus resulting in an increase in molecular weight. Typically, treatment of the proteinaceous molecules is performed under reaction conditions ensuring the removal and/or alteration of all post-translational modifications of a specific type to be analyzed that are comprised in the molecules in order to avoid the occurrence of any "intermediate molecules" where one or more modifications have been removed and/or altered but one or more others of the same type remain unchanged.

The removal and/or alteration of said specific post-translational modification results in an alteration of the physico-chemical properties of only the at least first subset of proteinaceous molecules compared to the remaining proteinaceous molecules such that this at least first subset can be identified during the subsequent re-fractionation step, since only this at least first subset will sort into a different fraction. All other proteinaceous molecules comprised in the sample will sort into the same fraction as during the initial fractionation step prior to removing/altering the post-translational modification.

Thus, for identifying and/or separating the at least first subset of proteinaceous molecules bearing this specific modification analyzed the results obtained in the initial and the re-fractionation step have to be compared (for example, comparing the specific patterns obtained after staining the gel used for electrophoretic protein separation).

In order to facilitate the sorting/separation process the fractions obtained after the initial fractionation step are preferably treated individually to removing/altering the post-translational modification before applying them to re-fractionation.

In further preferred embodiments of the invention, the method is used for the discrimination of different subsets of proteinaceous molecules comprising a specific post-translational modification. Particularly preferably, the method of the invention is used for discriminating phospho-proteins and/or -peptides, namely serine/threonine-phosphorylated (i.e. the phosphate is attached to a serine or a threonine amino acid residue) and tyrosine-phosphorylated proteins and/or peptides (i.e. the phosphate is attached to a tyrosine amino acid residue). Since these subsets of phospho-proteins and/or -peptides are regulated by different kinases and phosphatases, they have also been implicated in different cellular processes or signaling cascades.

Importantly, the two above subsets of phospho-proteins and/or -peptides also differ in their accessibility to chemical treatment with regard to the removal and/or alteration of post-translational modifications. For example, serine- and threonine-phosphorylated proteinaceous molecules are accessible to the chemical removal of the phosphate group via β-elimination (i.e., a type of elimination reaction well established in the art, wherein atoms or atom groups are removed from two adjacent atoms of the substrate while forming a 1t bond), whereas tryrosine-phosphorylated proteinaceous molecules are not. This difference provides a basis for distinguishing these to subsets of phospho-proteins and/or -peptides.

Thus, in preferred embodiments of the invention, the method is configured to allow the discrimination between serine- and threonine-phosphorylated and tyrosine-phosphorylated proteinaceous molecules, respectively. Based on the above-mentioned accessibilty to chemical treatment it is particularly preferred that the at least first subset of proteinaceous molecules comprises serine- and threonine-phosphorylated proteinaceous molecules. In other words, following the initial fractionation step the first subset to be enriched and/or separated from tyrosine-phosphorylated and unphosphorylated proteinaceous molecules comprises serine- and threonine-phosphorylated proteinaceous molecules.

Preferably, in any embodiments of the invention relating to the analysis of phospho-proteins and/or -peptides the phosphate-group is removed chemically via β-elimination. However, it is also possible to remove the phosphate-group, for example, enzymatically by means of specific or non-specific phosphatases.

To specifically remove the phosphate moieties from phospho-serine and phospho-threonine amino acid residues, β-elimination is typically induced by base hydrolysis. In order to avoid any adverse effects on the side chains of cysteine and methionine residues, the samples may first be treated with performic acid, resulting in the oxidation, and thus inactivation of these residues. A typical reaction mixture for performing β-elimination comprises H₂O, dimethyl sulfoxide, acetonitrile, 250 mM EDTA (pH 8.0), and 5 M NaOH.. The samples are incubated for 1 h at 55°C under a N₂ atmosphere, cooled to room temperature, and quenched by neutralizing with acetic acid. Removing the phosphate-group via β-elimination results in forming an unnatural amino acid (e.g., dehydro-alanine, dehydro-2-amino butyric acid).

After subjecting the individual fractions obtained after the initial fractionation step to the above treatment, they are re-fractionated, typically by applying the same fractionation method, preferably isoelectric focusing.

The removal and/or alteration of the phosphate group specifically from phospho-serine and phospho-threonine amino acid residues results in an alteration of the physico-chemical properties of only those subset of proteinaceous molecules (i.e. the at least first subset) comprising such modified amino acid residues such that they can be identified during the subsequent re-fractionation step, since only this subset will sort into a different fraction. All other proteinaceous molecules comprised in the sample including tyrosine-phosphorylated and - of course - unphosphorylated proteinaceous molecules will sort into the same fraction as during the initial fractionation step prior to removing/altering the post-translational modification. Thus, using the above-described method serine- and threonine-phosphorylated proteinaceous molecules can be selectively enriched from a complex sample by comparing the results of the initial and the re-fractionation step performed.

In a further typical embodiment, the inventive method further comprises the selective enrichment and/or separation of at least a second subset of proteinaceous molecules from the subset of proteinaceous molecules remaining after separation of the at least first subset following the re-fractionation step, as described above. To accomplish this goal, the remaining subset of proteinaceous molecules is subjected to another cycle of fractionation, removing or altering a specific post-translational modification, and re-fractionation, wherein the modification from at least a second subset of the proteinaceous molecules is removed or altered.

Preferably, the post-translational modification of the at least second subset of the proteinaceous molecules that is to be removed or altered is a phosphate-group. However, it is also within the scope of the present invention to analyze at this stage any other type of modification than that analyzed during the first cycle of fractionation, removing or altering a specific post-translational modification, and re-fractionation. For example, it is possible to analyze in the first cycle phospho-proteins and/or -peptides and in the second cycle ubiquitinylated proteins and/or peptides.

Particularly preferably, the at least second subset of proteinaceous molecules is tyrosine-phosphorylated proteinaceous molecules. Since phosphorylated tyrosine residues are not accessible to chemical cleavage via β-elimination, it is preferred to remove the phosphate group enzymatically. Enzymatic removal may be achieved via phosphatases, particularly via alkaline phosphatases (for the non-specific removal of phosphate-groups) or via protein tyrosine phosphatases (for specifically dephosphorylating phospho-tyrosine residues). Such phosphatases are commercially available.

By performing a second re-fractionation step the tyrosine-phosphorylated proteinaceous molecules can be separated from their unphosphorylated counterparts based on the same principle as described above. The remaining subset of proteinaceous molecules may optionally be subjected to a third, forth, and so forth cycle of fractionation, removing/altering a post-translational modification, and re-fractionation.

In a further embodiment, the invention relates to the use of a method, as described herein, for the qualitative and/or quantitative determination of phospho-proteins/peptides, particularly in connection with proteomic studies.

In preferred embodiments, the determination is performed via mass spectrometry, an analytical technique used to measure the mass-to-charge ratio of ions. Importantly, at least for phospho-proteins and/or -peptides mass spectrometric analysis is facilitated by the removal of the unstable phosphate-group but leaving behind a unique unnatural amino acid for the serine- and threonine-phosphorylated species.

While the above invention has been described with respect to some of its preferred embodiments, this is in no way to limit the scope of the invention. The person skilled in the art is clearly aware of further embodiments and alterations to the previously described embodiments that are still within the scope of the present invention.

### EXAMPLES

### Example 1

Commercially available instrumentation such as the IPGPhor IEF unit (Amersham-Pharmacia, Piscataway, NJ, USA) was used for electrophoretic separation of the protein and/or peptide samples employed according to their isoelectric point. Chemical or enzymatic digestion of the proteins into peptides was optionally performed following established standard protocols.

To specifically remove the phosphate moieties from phosphoserine and phosphothreonine amino acid residues, base hydrolysis was used to induce β-elimination. In order to avoid any adverse effects on the side chains of cysteine and methionine residues, the samples were first treated with performic acid, resulting in the oxidation of these residues, thereby inactivating them (Goshe, M.B. et al.(2001) Anal. Chem. 73, 2578-2586).

The β-elimination reaction mixture used was as follows: H₂O, dimethyl sulfoxide, acetonitrile, 250 mM EDTA (pH 8.0), and 5 M NaOH. All solvents were degassed with N₂ before and after preparation of the reaction mixture.

The β-elimination reaction mixture was added to the lyophilized protein fractions isolated from the gel after isoelectric focusing . The samples were incubated for 1 h at 55°C under a N₂ atmosphere, cooled to room temperature, and the reaction quenched by neutralizing with acetic acid (Goshe, M.B. et al.(2001), *supra*)

Commercially available alkaline phosphates were used to dephosphorylate phospho-tyrosine amino acid residues.

By treating the protein and/or peptide samples in the above-described manner and subsequent electrophoretic separation of the respective fractions according to their isoelectric point serine/threonine-phosphorylated proteins and/or peptides as well as tyrosine-phosphorylated proteins and/or peptides could be selectively enriched and separated.

## Claims

1. Method for the selective enrichment and/or separation of post-translationally modified proteinaceous molecules, comprising at least the steps of:
(a) fractionating the proteinaceous molecules;
(b) removing or altering a specific post-translational modification from at least a first subset of the proteinaceous molecules;
(c) re-fractionating the proteinaceous molecules obtained in step (b);
(d) comparing the fractionation patterns obtained in steps (a) and (c); and
(e) analyzing the at least first subset of proteinaceous molecules modified in step
(b) based on the results obtained in step (d).

2. The method of claim 1, further comprising cleaving the proteinaceous molecules into peptides prior to subjecting them to fractionation.

3. The method of claim 1 or 2, wherein the fractionation/re-fractionation is performed by isoelectric focusing.

4. The method of any of claims 1 to 3, wherein the post-translational modification of the at least first subset of the proteinaceous molecules that is to be removed or altered is a phosphate-group.

5. The method of claim 4, wherein the at least first subset of proteinaceous molecules comprises serine- and threonine-phosphorylated proteinaceous molecules.

6. The method of claim 4 or 5, wherein the phosphate-group is removed chemically via β-elimination.

7. The method of any of claims 1 to 6, wherein after performing step (e) the remaining subset/s of proteinaceous molecules is/are subjected to another cycle of steps (a) to (e), and wherein step (b) comprises removing or altering a specific post-translational modification from at least a second subset of the proteinaceous molecules.

8. The method of claim 7, wherein the post-translational modification of the at least second subset of the proteinaceous molecules that is to be removed or altered is a phosphate-group.

9. The method of claims 7 or 8, wherein the at least second subset of proteinaceous molecules comprises tyrosine-phosphorylated proteinaceous molecules.

10. The method of any of claims 7 to 9, wherein the phosphate-group is removed enzymatically via phosphatases.

11. Use of a method of any of claims 1 to 10 for the qualitative and/or quantitative determination of phospho-proteins and/or -peptides.

12. The use of claim 11, wherein the determination is performed via mass spectrometry.
